Europäisches Patentamt

European Patent Office

Office européen des brevets

(19)

(11) Publication number: **0 169 907**

**A1**

(12) **EUROPEAN PATENT APPLICATION**

published in accordance with Art. 158(3) EPC

(21) Application number: **85900730.4**

(22) Date of filing: **09.01.85**

Data of the international application taken as a basis:

(86) International application number:
**PCT/JP85/00004**

(87) International publication number:
**WO85/03086 (18.07.85 85/16)**

(51) Int. Cl.⁴: **C 12 P 21/00**
**C 12 N 15/00, C 07 K 15/26**
**A 61 K 45/02**
**///(C12P21/00, C12R1:91),**
**(C12N15/00, C12R1:91)**

(30) Priority: **09.01.84 JP 1649/84**

(43) Date of publication of application:
**05.02.86 Bulletin 86/6**

(84) Designated Contracting States:
**AT BE CH DE FR GB LI LU NL SE**

(71) Applicant: **Suntory Limited**
**1-40 Dojimahama 2-chome Kita-ku**
**Osaka-shi Osaka-fu 530(JP)**

(72) Inventor: **NAGATA, Shigekazu**
**3-305, 24-62, Tamagawa 2-chome**
**Ohta-ku Tokyo 146(JP)**

(72) Inventor: **FUKUNAGA, Rikirou**
**8 Eminanso 22, Tanakaohkubocho**
**Ukyo-ku, Kyoto-shi Kyoto 615(JP)**

(72) Inventor: **SOUKAWA, Yoshihiro 302, Poruto-do-okazaki**
**140 Okazakienshojicho Sakyo-ku, Kyoto-shi**
**Kyoto 606(JP)**

(72) Inventor: **KAJIRO, Yoshito**
**22-10, Shakujiimachi 6-chome**
**Nerima-ku Tokyo 177(JP)**

(74) Representative: **Hoffmann, Klaus, Dr. rer. nat. et al,**
**Hoffmann . Eitle & Partner Patentanwälte**
**Arabellastrasse 4**
**D-8000 München 81(DE)**

(54) **PROCESS FOR PREPARING SUGAR-ADDED POLYPEPTIDES.**

(57) A process for preparing sugar-added polypeptides, particularly human gamma interferon, which comprises culturing mammal cells transformed by DNA of bovine papilloma virus origin in which a polypeptide gene, particularly human gamma interferon gene, is incorporated so as to constantly function under the control of Primates virus promoter, then collecting produced sugar-added polypeptide from the culture liquor.

EP 0 169 907 A1

./...

FIG. 4

SPECIFICATION

PROCESS FOR PRODUCING SUGAR-CONTAINING POLYPEPTIDE

Technical Field

The present invention relates to a process for producing a sugar-containing polypeptide. More particularly, the invention relates to a process for producing a sugar-containing polypeptide comprising the steps of transforming a mammalian animal cell by a bovine papilloma virus derived DNA in which a gene coding for the desired polypeptide has been incorporated in such a way as to ensure consistent expression of said polypeptide under the control of a primate viral promoter, cultivating the transformed animal cell, and recovering the desired polypeptide from the culture supernatant. The invention also relates to a culture composition containing said polypeptide.

Background Art

Many attempts have recently been made to produce useful and physiologically active substances by recombinant DNA technology (hereunder referred to as rDNA technology). It can be said that the fundamental techniques for rDNA technology have reached the stage of completion on a laboratory scale. However, there remain many problems that have to be solved before the rDNA technology can be used on a practical basis. For example, the development of techniques for producing glycoprotein which frequently occurs in natural physiologically active substances is far from the stage of completion. In the production of useful substances by rDNA technology, procaryotic cells such as Escherichia coli and Bacillus subtilis are extensively used as host cells, but these microbial hosts are not suitable for the production of glycoprotein. Therefore, an increasing amount of research has been directed to the production of glycoprotein by using eucaryotic cells (e.g. yeasts and animal cells) capable of producing glycoproteins as the host, but no practical host-vector system has been established to data. For instance, attempts have been made to produce the desired foreign protein or polypeptide by culturing monkey cells (e.g. COS cells) into which a heterologous DNA coding for the protein has been introduced carried on a vector which is Simian Virus 40 (hereunder abbreviated as SV40) derived from rhesus monkey (see Hamer, D. H. et al., Nature, 281: 35 - 40, 1979; Gruss, P. et al., Proc. Natl. Acad. Sci., USA, 78: 133 - 137, 1981; Gray, P. W. et al., Nature, 295: 503 - 508, 1982; and Devos, R. et al., Nucl. Acid. Res., 10: 2487 - 2501, 1982). However, the previous attempts have the following disadvantages: (1) the size of the heterologous DNA to be inserted into SV40 and the type of host cells used are limited; (2) it is difficult to transform the host cells; (3) even transformed host cells may be killed by the introduced DNA which will act as a "virus"; and (4) for these reasons, the expression of the desired foreign protein encoded by the inserted gene is inadequate. Some of these defects have partly been overcome by the development of new vectors (Lowy, D. R. et al., Nature 287: 72 - 74, 1980 and Sarver, N., Mol. Cellular Biol., 1: 486 - 496, 1981), but they are not yet sufficient to be used in the practical production of the desired glycoprotein.

The present inventors have therefore made extensive studies in order to establish a host-vector system that is free from the above described defects of the conventional techniques and which is expected to be suitable for commercial use. As a result, the inventors have found that if mouse breast cancer cells are used as host cells and if a DNA comprising a bovine papilloma virus gene capable of expressing the desired foreign gene under the control of an SV40 early promoter is used as a vector, high yields of glycoprotein (human gamma-interferon, in particular) can be produced with consistent results. The present invention has been accomplished on the basis of this finding.

Disclosure of the Invention

The present invention provides a process for producing a sugar-containing polypeptide comprising the steps of transforming a mammalian animal cell by a bovine papilloma virus derived DNA in which a gene coding for the desired polypeptide has been incorporated in such a way as to ensure consistent expressing of said polypeptide under the control of a primate viral promoter, cultivating the transformed animal cell, and recovering the desired polypeptide from the culture supernatant.

The term "sugar-containing polypeptide" used in this specification means that a polypeptide that will occur as a glycoprotein can actually be produced by the method of the present invention as a substance having a suger portion.

A particularly preferred primate virus promoter for use in the invention is an SV40 early promoter.

The method of the present invention is particularly useful for producing a sugar-containing human interferon (especially human gamma-interferon) in mammalian animal cells using a human interferon gene as a polypeptide gene. It is expected that the method of the invention is also applicable to the production of other polypeptides having a sugar portion.

When producing a human gamma-interferon by the method of the present invention, a human gamma-interferon gene prepared by using, as a template, the corresponding messenger RNA from human spleen cells or human lymphocytes may advantageously be used as a gene coding for the intended human gamma-interferon.

Details of the DNA derived from bovine papilloma virus into which the polypeptide gene is incorporated, as well as the method of conducting such incorporation are found in the Example to be shown later in this specification, but it should be understood that said Example is not to be taken as limiting the invention.

Any mammalian animal cells that can be transformed by the bovine papilloma virus derived DNA may be used in the method of the present invention, and mouse derived cells are one example.

Brief Description of the Drawings

Fig. 1 shows the amino acid sequence of the neighborhood of the carboxyl terminal of human gamma-interferon (hIFN-γ), the corresponding base sequence, and the base sequence of the chemically synthesized DNA probe (as indicated in the lower box) used for separating hIFN-γ mRNA;

Fig. 2 shows the restriction enzyme mapping of a cloned hIFN-γ cDNA, as well as a schematic presentation of the method of constructing plasmid pIFγ having a full length of hIFN-γ gene from two hIFN-γ cDNA fragments (pγ-1002 and pγ-1018) obtained by the Okayama-Berg method using pγ-300 DNA as a probe;

Fig. 3 shows schematically the method of constructing expression vector pdKCR-IFN for expressing hIFN-γ gene in COS-1 cells;

Fig. 4 shows schematically the method of constructing expression vector pFNγ for expressing hIFN-γ gene in mouse breast cancer cells;

Fig. 5 shows the relationship between cell population and antiviral activity as observed during the cultivation of transformed Fγ56 cells; and

Fig. 6 is a graph showing the effect of the concentration of fetal calf serum (FCS) in a culture medium on the production of hIFN-γ.

Best Mode of Carrying out the Invention

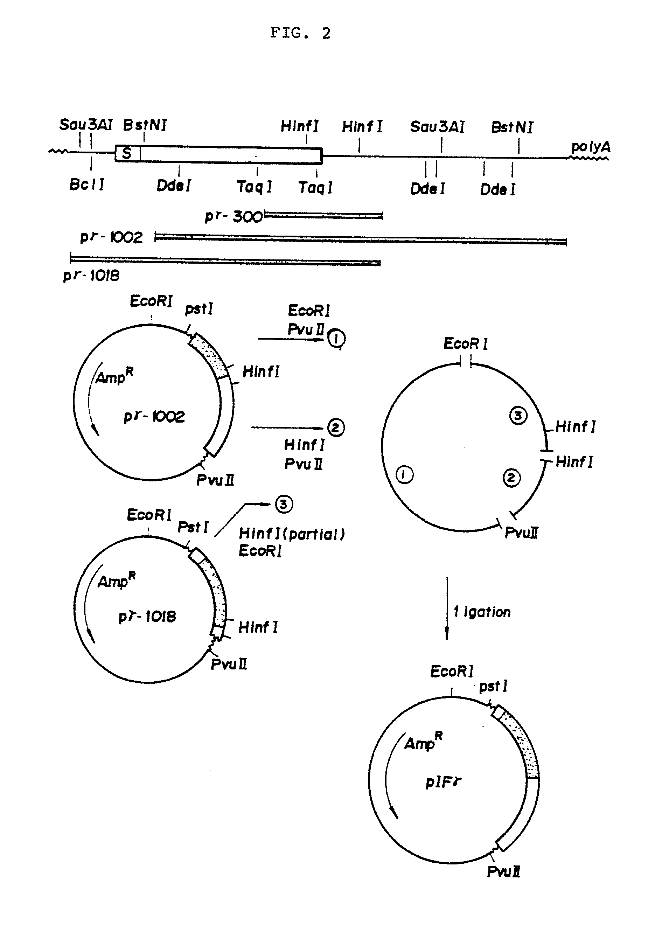

The gene of a human gamma-interferon (hereunder abbreviated as hIFN-γ) which is one of the glycoproteins that can be produced by the method of the present invention has already been cloned by Gray, P. W. et al. (Nature, 295: 503 - 508, 1982) and Devos, R. et al. (Nucl. Acid. Res.; 10: 2487 - 2501, 1982) who also determined the DNA sequence of this gene. The present inventors independently obtained the hIFN-γ gene from cloned cDNA fragments that were prepared by using, as a template, the mRNA produced by human spleen cells. First, human spleen cells are cultured in the presence of SEA (Staphylococcal enterotoxin A). From the resulting mRNA, a cDNA bank may be prepared by the commonly used method of Hoeijmokers et al. (Hoeijmokers, J. H. J. et al., Gene, 8: 391 - 417, 1980). From this cDNA bank, hIFN-γ cDNA fragments are obtained by using a chemically synthesized oligonucleotide as a probe. Then, one of the cDNA fragments is used as a probe for again isolating hIFN-γ cDNA fragments from a cDNA bank prepared by the Okayama-Berg method (Okayama, H. & Berg, P., Mol. Cell. Biol. 2: 161 - 170, 1982). The so isolated hIFN-γ cDNA fragments may be properly conjugated together to form a full length of hIFN-γ gene (Hu-IFN-γ cDNA). For details of these procedures, see Fig. 2.

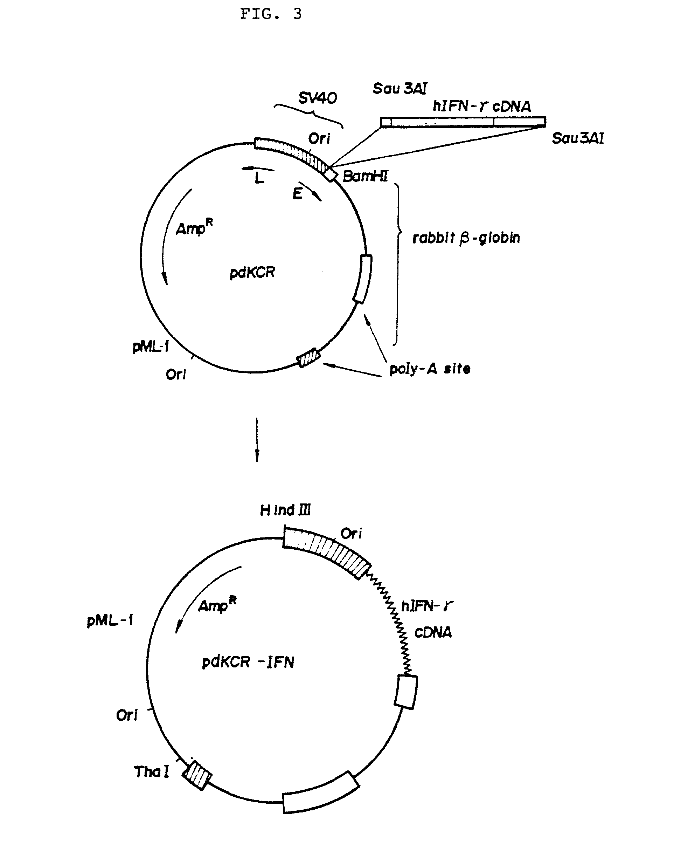

The identity of the so obtained hIFN-γ gene may be confirmed by the following procedures. First, it is inserted at BamHI site (see Fig. 3) on a hybrid plasmid pdKCR containing a rabbit β-globin gene, SV40 DNA and pBR322 DNA derivative (pML-1). Such hybrid plasmid was received from Dr. Breathnach of the INSERM Institute at Strasbourg, France. The inserted gene is used to transform monkey derived COS-1 cells (received from Dr. Gluzman at the Cold Spring Harbor Laboratory, U.S.A.) by the calcium phosphate method (Wigler, M. et al., Cell 14: 725 - 731, 1978). Then, the transformant cells are cultured and the antiviral activity in the culture supernatant is assayed by the method of Yip et al. (Yip, Y. K. et al., Proc. Natl. Acad. Sci., USA 78: 1601 - 1605, 1981). Antiviral activity will be observed which is not lost by treatment with an anti-interferon α or anti-interferon β antibody but is lost by treatment with anti-hIFN-γ antibody. This result indicates that the obtained gene is hIFN-γ gene. However, the ability of the transformant cells to produce hIFN-γ is transient and a method using them is not suitable for use in the practical production of hIFN-γ.

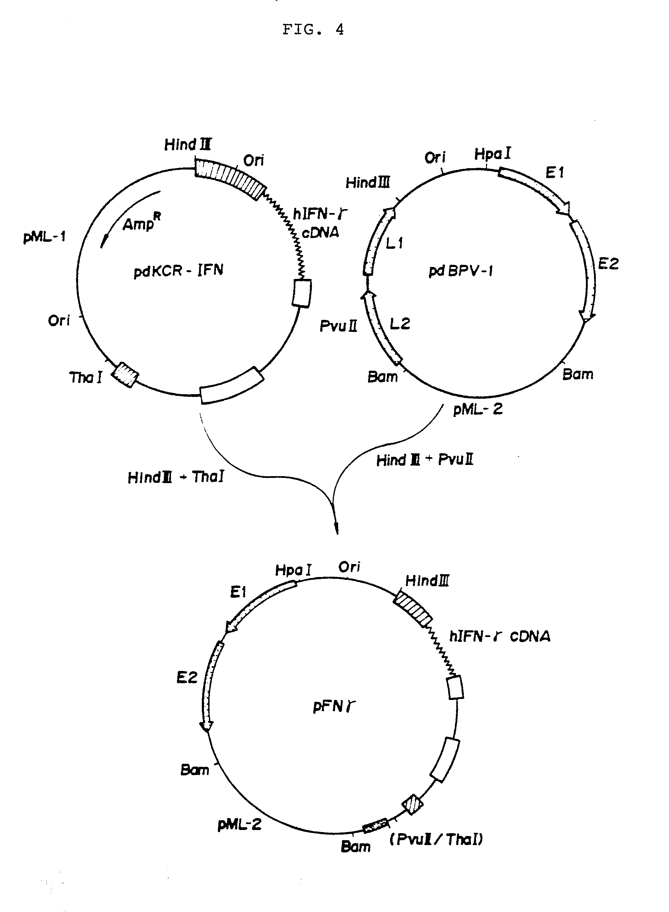

The present inventors therefore devised a more practically feasible method. First, a DNA fragment comprising the previously obtained hIFN-γ gene is inserted at Hind III-PvuII site on another plasmid pdBPV-1 (a hybrid plasmid consisting of bovine papilloma virus DNA and pLM-2, a derivative from pBR322 DNA; said plasmid was received from Dr. Peter M. Howley of the National Cancer Institute, Bethesda, U.S.A. and has been deposited with the American Type Culture Collection under ATCC No. 37134 in E. coli HB 101 (ACTT No. 33694)). After deleting the pML-2 region originating from pBR322 the plasmid is used to transform mouse C127I cells (available from the American Type Culture Collection; deposited as ATCC No. CRL 1616) by the calcium phosphate method (ibid) (see Fig. 4). Subsequently, the transformed cells are cultured and the amount of hIFN-γ secreted into the culture supernatant is assayed in terms of the antiviral activity. In accordance with the inventors' results, the amounts of hIFN-γ produced by different strains of transformant were varied, but at least 80% of the transformants exhibited an antiviral activity. It is interesting to note that the culture supernatant of several of these transformants yielded a significantly high antiviral activity (ca. $4.5 \times 10^5$ units/ml). Not a single recombinant gene expressing in an animal cell host has yet been found to exhibit such a high productivity. The present inventors confirmed by the Southern method (Southern hybridization: Southern, E. M., J. Mol. Biol., 98: 503, 1975) that the plasmid (pFNγ: see Fig. 4) constructed by the present invention can be present in the host cell in large copy number (20 - 50 copies per cell) and can be replicated consistently.

There will be no doubt as to the presence of a sugar chain attached to the hIFN-γ polypeptide produced by the transformant in accordance with the present invention, because said active substance is adsorbed on ConA Sepharose, and estimation by SDS polyacrylamide gel electrophoresis shows that said substance has a molecular weight of 20,000 to 25,000 (if it were a pure polypeptide, the molecular weight would be approximately 17,000).

The method of producing glycoprotein using the host-vector system defined in the present invention has the following advantages that make the method highly suitable for use in commercial operations: the plasmid into which the desired foreign gene has been incorporated in such a manner that it can be consistently expressed has a large copy number and can be consistently replicated as an episomic DNA; the intended substance is secreted as a glycoprotein in high yield in a culture medium and is therefore suitable for production by continuous cultivation.

The method of the present invention is described above with reference to the case where the desired substance is human gamma-interferon (hIFN-γ). However, it will be readily understood by those skilled in the art that the method is also applicable to the production of other glycoproteins such as human beta-interferon and interferons derived from animals other than humans, as well as lympho-kines such as interleukines 1, 2 and 3, TNF (tumor necrosis factor) and CSF (colony stimulating factor), and hormones containing a sugar chain, and even antibodies. The host cells that can be used in the present invention are not limited to mouse breast cancer cells, and any other established animal cell lines that are capable of accepting the plasmid DNA may be used.

The present invention is hereunder described in greater detail by reference to the Example which is by no means intended as limiting the invention. The plasmid and cells used in the method of the present invention are available from the sources already described.

Example

(1) Cloning of human gamma-interferon (hIFN-γ) gene:

Human spleen cells ($1.0 \times 10^{10}$) were cultured for 60 hours at 37°C in RPMI 1640 medium containing 5% bovine serum and 0.02 µg/ml of SEA (Staphylococcal enterotoxin A) and RNA (4 mg) was extracted by the method of Chirgwin et al. (Chirgwin, J. M. et al., Biochemistry 18: 5294 - 5299, 1979). Then, poly-A RNA was separated by oligo-dT-cellulose column chromatography (Aviv, H. et al., Proc. Natl. Acad. Sci., USA, 69: 1408 - 1412, 1972). Subsequently, using RPS-40 rotor (Hitachi), centrifugation (26,000 rpm x 19 hours) was performed at 4°C on 5 - 25% sucrose linear density gradient containing 50 mM tris-HCl (pH: 7.5), 0.2M NaCl, 0.1% sodium N-lauroylsarcosine and 1 mM EDTA. Each of the fractions (0.2 ml) was concentrated by ethanol precipitation, and one twentieth of the concentrate was injected into an egg of Xenopus oocytes so as to synthesize protein (see Gurdon, J. B., J. Molec. Biol., 80: 539 - 551, 1975). Antiviral activity was observed in the fractions corresponding to 15S, which were collected and used as hIFN-γ mRNA containing fractions in the subsequent synthesis of hIFN-γ cDNA.

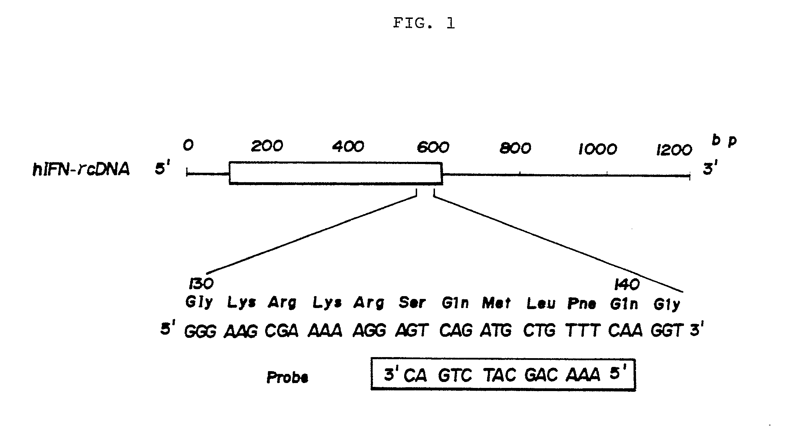

From the mRNA (20 µg) purified by centrifugation on sucrose density gradient, a double-stranded cDNA (0.24 μg) joined to pBR322 by the G-C tail method was prepared in accordance with the method of Hoeijmakers et al. (Hoeijmakers, J. H. J. et al., Gene 8: 391 - 417, 1980). This DNA was used to transform E. coli so as to obtain 4,000 clones of transformant having tetracycline resistance. In order to select the clones having hIFN-γ cNDA, colony hybridization was performed using $^{32}$P labelled (14 mer) nucleotide as a probe (see Fig. 1). The base sequence of this DNA probe was designed and chemically synthesized by reference to the DNA sequence of hIFN-γ shown by Gray et al. (Gray, P. W. et al., Nature 295: 503 - 508, 1982) (see Fig. 1). Said labelled nuclotide probe was purchased from MS Instruments.

Four positive clones were selected by colony hybridization of the 4,000 clones of tetracycline-resistance transformant. Subsequently, plasmid was separated from each of these positive clones and analyzed by cleavage with restriction enzymes on the basis of the base sequence data reported by Gray and Devos (ibid). The analysis showed that the largest hIFN-γ cDNA fragment obtained consisted of 300 bp while a full length of hIFN-γ DNA is required to consist of at least 500 bp. This hIFN-γ cDNA having about 300 nucleotides was therefore named pγ300 and used in the subsequent separation of hIFN-γ cDNA from a cDNA bank.

The cDNA bank in this separation step was prepared by the Okayama-Berg method (Okayama, H & Berg, P., Mol. Cell. Biol. 2: 161 - 170, 1982) from poly-A RNA (3 μg) obtained by centrifugation on sucrose density gradient. Colony hybridization was performed by the same method as described above using pγ300 as a probe. Two clones having hIFN-γ cDNA fragments were selected from 6,000 strains of transformed E. coli, and respectively named pγ-1002 and pγ-1018. The plasmid of each of these clones was analyzed by the same method as described above, and as shown in Fig. 2, both cDNA fragments were found to be larger than pγ300. However, pγ-1002 lacked a 5' end portion while pγ-1018 lacked a 3' end portion. Thus, as shown in Fig. 2,

plasmid pIFγ having a full length of hIFN-γ gene was constructed by joining EcoRI-PvuII fragment (1) (including Amp$^r$), HinfI-PvuII fragment (2) each from pγ-1002 and EcoRI-HinfI fragment (3) (not including Amp$^r$) from pγ-1018 by means of T$_4$DNA ligase. The restriction enzyme mapping of the complete cDNA region, namely hIFN-γ gene region, prepared from the pIFγ plasmid introduced into E. coli was found to be identical with the restriction enzyme mapping proposed by Gray et al. (ibid).

(2) Expression of hIFN-γ gene in monkey COS-1 cells:

The following experiment was conducted in order to check whether the hIFN-γ gene obtained in (1) above would be expressed in animal cells and produce hIFN-γ polypeptide.

The expression vector plasmid pdKCR used was received from Dr. Breathnach of the INSERM Institute, Strasbourg, France. The plasmid pdKCR was composed of a DNA fragment in the initiator region of SV40 , rabbit β-globin gene and a DNA fragment containing Amp$^r$ (ampicillin-resistant) gene derived from pBR322  it was so designed that when a heterologous DNA was inserted at BamHI  site, it would be expressed in COS-1 cells under the control of SV40  early promoter (see Fig. 3). Thus, the present inventors constructed an expression vector pdKCR-IFN by first preparing Sau3A DNA fragment of 850 bp by cleaving the plasmid pIFγ, which contains hIFN-γ gene and was obtained in (1) above, with a restriction enzyme Sau3AI and then inserting the resulting Sau3A DNA fragment into pdKCR plasmid at a BamHI site (Fig. 3). Subsequently, this vector was treated with restriction enzyme, HhaI, to remove the pBR322  derived DNA (pML$_1$ region), and thereafter, COS-1 cells (received from Dr. Gluzman of the Cold Spring Harbor Laboratory, U.S.A.) were transformed by the calcium phosphate method (Wigler, M. et al., Cell 14: 725 - 731, 1978). A control was prepared by introducing pdKCR free of hIFN-γ into COS-1 cells.

Two groups of COS-1 cells, one transformed with plasmid pdKCR-IFN and the other by pdKCR, were cultured for 90 hours at 37°C in DMEM medium (Dulbecco's modified Eagle medium "Nissui", purchased from Nissui Seiyaku) containing 10% calf serum and the antiviral activity of the culture supernatant was determined. Antiviral activity that would be lost by treatment with anti-gamma-interferon antibody was observed in only the culture supernatant of the cells that had been transformed by pdKCR-IFN (see Table 1). It was therefore confirmed that the pIFγ obtained in (1) above did have the hIFN-γ gene.

### Table 1

Identification of COS-1 cell produced interferon

| Plasmid | Control | Anti-interferon α/β serum | Anti-interferon γ serum |
|---|---|---|---|
| pdKCR | <10 | n.t. | n.t. |
| pdKCR-IFN | 1.250 | 1.150 | 16 |

n.t.: not tested.

As Table 1 shows, the COS-1 cells transformed by pdKCR-IFN plasmid were able to produce hIFN-γ but the transformed cells died very soon, resulting in only the transient production of hIFN-γ by this host-vector system. Therefore, the inventors proceeded with their experiment in order to construct cells capable of consistently producing high yield of hIFN-γ.

(3) Construction of cells capable of consistent production of hIFN-γ in high yield:

Plasmid pdBPV-1 received from Dr. Peter M. Howley of National Cancer Institute, Bethesda, U.S.A. was used as a plasmid vector having high copy number and being capable of consistent replication. The pdBPV-1 is a hybrid plasmid that has bovine papilloma virus DNA and pBR322 derived pML-2 vector DNA and which is capable of replication in mouse cells while maintaining high copy number (50 - 100 per cell).

Plasmid pFNγ capable of consistent production of hIFN-γ in mouse cells while maintaining high copy number was constructed by the procedures shown in Fig. 4. Stated specifically, pdBPV-1 was treated with HindIII and PvuII to remove the late protein region of bovine papilloma virus

(all of $L_1$ and a part of $L_2$ in Fig. 4), and the depleted region was filled with a HindIII-ThaI DNA fragment containing the hIFN-γ gene region of pdKCR-IFN previously obtained. Then, this plasmid pFNγ was treated with BamHI to remove the pML-2 region originated from pBR322, and was thereafter used to transform mouse Cl27I cells by the calcium phosphate method described before. As a control, BamHI treated plasmid pdBPV-1 was also used to transform these cells by the same procedure. The cells were cultured in DMEM medium (with 10% calf serum) that was replaced with a fresh medium every third day. Transformed strains observed at 16 days as focuses were established as cell lines.

The frequency of transformation of Cl27I cells by BamHI treated pFNγ DNA was about one fifteenth of the frequency of transformation by BamHI treated pdBPV-1. However, 33 transformed strains per 10 μg of DNA was obtained. A total of 56 strains were obtained by transformation with pFNγ DNA. The production of antiviral substance (i.e. hIFN-γ) in the culture supernatant of each of these transformants was determined by the same method as described in (2) above; the culture supernatant of 45 transformants had measurable antiviral activities in varying degrees.

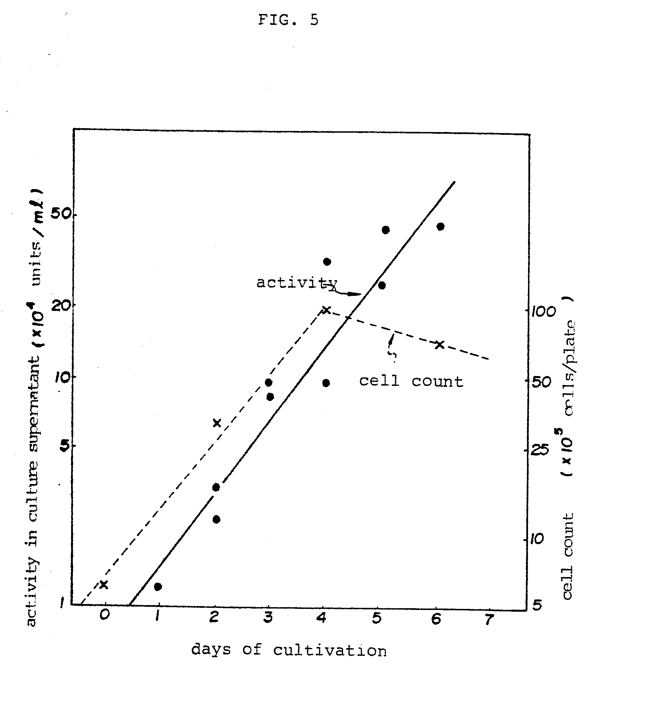

The ability of hIFN-γ production by Fγ56 which was one of the transformants that exhibited high activity was further examined by the following procedures. Cells (5 x $10^5$) were plated in DMEM medium (in Petri dish with 5 cm²) containing 10% calf serum and cultured at 37°C. A cell count was conducted every other day while the interferon activity (antiviral activity) was measured daily (see Fig. 5). As is clear from Fig. 5, the antiviral activity increased until the 4th day of cultivation in proportion to the cell count, and thereafter, the antiviral activity continued to increase after the cell count substantially reached a plateau. At 6 days of the cultivation, the antiviral activity reached a surprisingly high level (5 x $10^5$ units/ml).

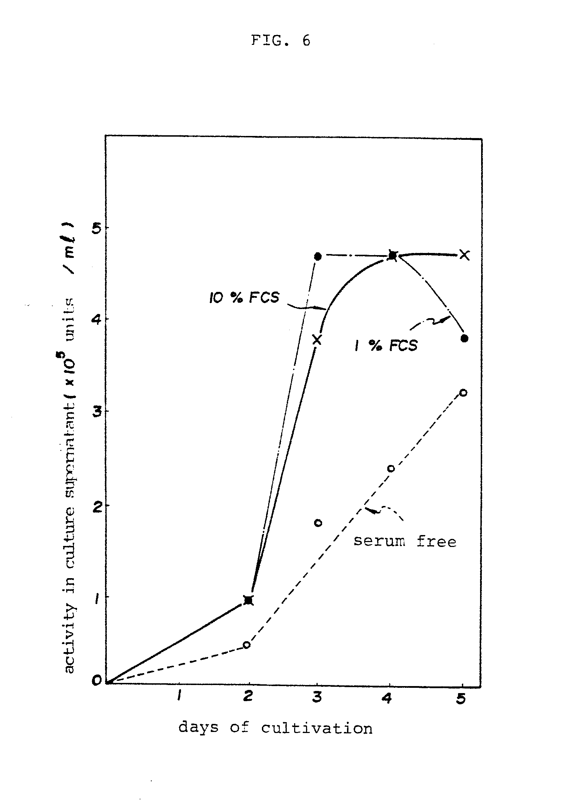

The effect of serum concentration in culture medium on the production of hIFN-γ was also examined.  The trans-
formant Fγ56 was cultured under the same conditions as used
above and at a time when no further increase in cell count
would occur (i.e., after approximately 4 days of cultivation), the medium was replaced by serum free DMEM medium,
DMEM medium containing 1% calf serum or DMEM medium containing 10% calf serum, and additional cultivation was conducted
on each of the media at 37°C.  The results of determination
of antiviral activity in each of the media, conducted daily,
are shown in Fig. 6.  For the first three days of cultivation, both calf serum containing media exhibited a similar
profile of increase in antiviral activity.  The medium
containing 10% of calf serum attained an antiviral activity
level of 4.5 x $10^5$ units/ml at 3 days while the medium
containing 1% of calf serum attained the same level at 4
days.  The interferon activity in the serum free medium
increased slowly with the progress of cultivation and
reached a level of 3 x $10^5$ units/ml at 5 days.

The above results show that the plasmid constructed
by the present invention was stably maintained in the host
cell, and that a foreign gene inserted into the plasmid
was expressed very efficiently.  In other words, the above
results show the ability of the transformed cells to produce
such a foreign protein as the illustrated hIFN-γ consistently,
effieicntly and continuously, suggesting the applicability
of the host cells to practical production of foreign proteins.

(4) Partial purification of hIFN-γ and its physicochemical
     properties:

The Fγ56 cells obtained in (3) above were cultured
in a DMEM medium containing 10% calf serum.  When the cell
count had substantially reached a plateau, the cells were
transferred into 0.5 ml of MEM medium (Nissui) containing
methionine labelled with $^{35}S$ (100 μCi) and 10% of dialyzed
calf serum.  After continued culturing for 20 hours at 37°C,
supernatant (0.5 ml) was obtained by centrifugation and
mixed overnight with 25 μl of controlled pore glass
(CPG-00350, 120/200 mesh, Funakoshi Yakuhin) at 4°C.

Subsequently, the pore glass (hereunder CPG beads) was collected by centrifugation and washed thoroughly with PBS buffer (20 mM sodium phosphate buffer containing 150 mM of NaCl, pH: 7.4). Thereafter, the hIFN-γ adsorbed on the CPG beads was eluted with PBS buffer containing 0.4 M tetramethylammonium chloride. By this procedure, hIFN-γ could be recovered in a yield of more than 90%.

The eluate (0.3 ml) from the CPG beads was loaded onto 0.2 ml of ConA-Sepharose (Pharmacia Fine Chemicals) column, which was washed with 25 volumes of PBS buffer. The hIFN-γ adsorbed on the ConA-Sepharose was eluted by 0.1 M α-methyl-D-mannoside solution in a recovery yield of 46%. The eluate was collected as partially purified hIFN-γ and analyzed by electrophoresis through 15% SDS polyacrylamide gel using standard protein markers. As a result, the partially purified hIFN-γ was observed as a broad band of molecular weights ranging between 20,000 and 25,000. On the other hand, according to the report of Gray et al. (Gray, P. W. et al., Nature 295: 503 - 508, 1982), the polypeptide portion of hIFN-γ was composed of 146 amino acid residues and had an approximate molecular weight of 17,000.

These results, i.e., the fact that the desired protein (hIFN-γ) was adsorbed on ConA-Sepharose and that it had a molecular weight in the range of 20,000 - 25,000, strongly suggest that the hIFN-γ produced by FY56 cells is a protein to which a sugar chain has been attached.

The hIFN-γ gene cloned in the present invention contained a DNA region coding for a signal peptide (composed of 20 amino acid residues), but with the host cells C127I used in the Example, as suggested by Sarver et al. (Sarver, N. et al., Mol. Cell. Biol., 1: 486 - 496, 1981), the signal peptide is presumably processed and therefore the polypeptide portion of the product of the invention is composed of a mature polypeptide chain, or a polypeptide having an approximate molecular weight of 17,000.

CLAIMS

1.    A process for producing a sugar-containing polypeptide comprising the steps of transforming a mammalian animal cell by a bovine papilloma virus derived DNA in which a gene coding for the desired polypeptide has been incorporated so as to ensure consistent expression of said polypeptide under the control of primate viral promoter, cultivating the transformed animal cell, and recovering the desired polypeptide from the culture supernatant.

2.    A process according to Claim 1 wherein the primate virus is SV40.

3.    A process according to Claim 1 wherein the promoter is SV40 early promoter.

4.    A process according to Claim 1 wherein the polypeptide gene is a gene coding for a human interferon.

5.    A process according to Claim 1 wherein the polypeptide gene is human gamma-interferon gene.

6.    A process according to Claim 1 wherein the polypeptide gene is human gamma-interferon gene constructed by using a messenger RNA from a human spleen cell or human lymphocyte as the template.

7.    A process according to Claim 1 wherein the transformed mammalian animal cell is a mouse derived cell.

8.    A process according to Claim 1 wherein the sugar-containing polypeptide has an interferon activity.

9.    A process according to Claim 1 wherein the sugar-containing polypeptide is  human gamma-interferon.

10.    A process according to Claim 1 wherein the bovine papilloma virus derived DNA into which a polypeptide gene has been so incorporated that it can be expressed under the control of a primate virus promoter is a plasmid DNA denoted by pFNγ.

11.    A process according to Claim 1 wherein the mammalian animal cell transformed by the bovine papilloma virus derived DNA into which a polypeptide gene has been so incorporated that it can be expressed under the control of a primate virus promoter is denoted by FY56.

12.    A composition which comprise a sugar-containing human gamma-interferon obtained from culture supernatant of a mouse cell transformed with plasmid pFNγ.

13.    A composition according to Claim 12 wherein the mouse cell transformed with plasmid pFNγ is denoted by Fγ56.

$-\dfrac{1}{6}-$

FIG. 1

FIG. 2

FIG. 3

FIG. 4

FIG. 5

days of cultivation

FIG. 6

# INTERNATIONAL SEARCH REPORT

International Application No. PCT/JP85/00004 0169907

## I. CLASSIFICATION OF SUBJECT MATTER (if several classification symbols apply, indicate all) ³

According to International Patent Classification (IPC) or to both National Classification and IPC   Int.Cl⁴ C12P21/00,
C12N15/00, C07K15/26, A61K45/02, // (C12P21/00, C12R1:91)
(C12N15/00, C12R1:91)

## II. FIELDS SEARCHED

| Minimum Documentation Searched ⁴ | |
|---|---|
| Classification System | Classification Symbols |
| IPC | C12P 21/00, C12N 15/00, C07K 15/26, A61K 45/02 |

| Documentation Searched other than Minimum Documentation to the Extent that such Documents are Included in the Fields Searched ⁵ |
|---|
| |

## III. DOCUMENTS CONSIDERED TO BE RELEVANT ¹⁴

| Category* | Citation of Document, ¹⁶ with indication, where appropriate, of the relevant passages ¹⁷ | Relevant to Claim No ¹⁸ |
|---|---|---|
| A | Tanpakushitsu Kakusan Koso Rinji Zokan (Kumikae Idenshi no Saibo eno Donyu to Hatsugen), Vol. 28, No.14, December. 1983 (Tokyo) Ishii Shunsuke, Imamoto Fumio "Saibo ni Donyu sareta Kumikae Idenshi no Hatsugen", P.1480-1499, Especially see P.1494-1495 | 1 - 11 |
| A | Ishikawa Kunihiko Henshu "Bessatsu Tanpakushitsu Kakusan Koso Interferon Kenkyu no Shinpo" December 1, 1981 (01. 12. 81) Kyoritsu Shuppan Kabushiki Kaisha, P.69-80, Especially see P.73-77 | 12 - 13 |
| T | JP, A, 59-51792 (Biogen Naamloze Vennootschap) 26 March 1984 (26. 03. 84) Page 34, upper left column and upper right column. (Family nashi) | 1 - 13 |

* Special categories of cited documents: ¹⁵
  - "A" document defining the general state of the art which is not considered to be of particular relevance
  - "E" earlier document but published on or after the international filing date
  - "L" document which may throw doubts on priority claim(s) or which is cited to establish the publication date of another citation or other special reason (as specified)
  - "O" document referring to an oral disclosure, use, exhibition or other means
  - "P" document published prior to the international filing date but later than the priority date claimed
  - "T" later document published after the international filing date or priority date and not in conflict with the application but cited to understand the principle or theory underlying the invention
  - "X" document of particular relevance; the claimed invention cannot be considered novel or cannot be considered to involve an inventive step
  - "Y" document of particular relevance; the claimed invention cannot be considered to involve an inventive step when the document is combined with one or more other such documents, such combination being obvious to a person skilled in the art
  - "&" document member of the same patent family

## IV. CERTIFICATION

| Date of the Actual Completion of the International Search ² | Date of Mailing of this International Search Report ² |
|---|---|
| April 5, 1985   (05. 04. 85) | April 15, 1985   (15. 04. 85) |
| International Searching Authority ¹ | Signature of Authorized Officer ²⁰ |
| Japanese Patent Office | |

Form PCT/ISA/210 (second sheet) (October 1981)